# EUROPÄISCHE PATENTANMELDUNG

(11) **EP 1 982 604 A1**
(43) Veröffentlichungstag der Anmeldung: **22.10.2008**
(21) Anmeldenummer: 07008102.1
(22) Anmeldetag: 20.04.2007
(51) Int. Cl.: A23L 1/30, A23L 1/302, A23L 1/303, A61K 36/00

(54) **Nahrungsergänzungsmittel zum Ausgleich von Nährstoffmangel**

(71) Anmelder: Naturheilzentrum Allgäu, 87527 Sonthofen (DE)
(72) Erfinder: Herzog, Edmund, 87527 Sonthofen (DE)
(74) Vertreter: Hermann, Bettina Celia

(57) **Zusammenfassung**

Nahrungsergänzungsmittel, welches einen Gemüsebestandteil, einen Grasbestandteil, einen Fruchtbestandteil und ein Vitamin umfasst. Optional umfasst das Nahrungsergänzungsmittel weiterhin ein Antioxidationsmittel, und / oder ein Koenzym, und / oder einen Kräuterbestandteil, und / oder einen Fischbestandteil, und / oder Nussbestandteil, und / oder ein Mineral, und / oder ein Protein, und / oder eine Aminosäure, und / oder Zink-Citrat, und / oder Curcumin, und / oder L-Carnetin, und / oder Taurin. Weiterhin ein Verfahren zur Herstellung eines solchen Nahrungsergänzungsmittels sowie dessen Verwendung zur Herstellung eines Medikaments zur Verbesserung des allgemeinen Gesundheitszustands.

## Beschreibung

Die Erfindung betrifft ein Nahrungsergänzungsmittel, welches einen Gemüsebestandteil, einen Grasbestandteil, einen Fruchtbestandteil und ein Vitamin umfasst.

Nahrungsergänzungsmittel sind Lebensmittel, welche zu einer ausgewogenen Ernährung beitragen und einen oder mehrere Nährstoffe beispielsweise in konzentrierter Form enthalten.

Nahrungsergänzungsmittel dienen dem Ausgleich eines Defizits in der Versorgung mit wichtigen Nährstoffen. Aufgrund der verbesserten Versorgung mit Nährstoffen kommt es zu einer Verbesserung des allgemeinen Gesundheitszustands bzw. des Allgemeinbefindens. Die Einnahme von Nahrungsergänzungsmitteln stellt eine präventive Maßnahme zur Krankheitsvorbeugung dar, aber auch eine Maßnahme zur Unterstützung der Genesung bei Erkrankungen bzw. Mangelerscheinungen.

Typische Inhaltsstoffe von Nahrungsergänzungsmitteln sind Pflanzenstoffe, Mineralstoffe, Vitamine und / oder Antioxidantien. Als pflanzliche Bestandteile für die Zubereitung von Nahrungsergänzungsmitteln sind Gemüse-, Frucht-, und Kräuterbestandteile bekannt.

US 2006/0147563 A1 beschreibt ein Nahrungsergänzungsmittel, welches einen Frucht-, einen Gemüse- und einen Kräuterbestandteil sowie Mineralien und Vitamine umfasst.

Auch die Verwendung von Proteinen als Bestandteil von Nahrungsergänzungsmitteln ist im Stand der Technik beschrieben. WO 00/37087 A1 offenbart ein Nahrungsergänzungsmittel welches Frucht-, Gemüse- und Kräuterbestandteile sowie Vitamine, Mineralien und Proteine (z.B. Enzyme) umfasst.

Auch Grasbestandteile werden als Nahrungsergänzungsmittel verwendet, da Gräser besonders reich an Ballaststoffen sind und die grünen Teile der Gräser einen hohen Anteil an Chlorophyll enthalten.

Zusammensetzungen auf Basis von Grasbestandteilen werden auch zur äußeren Anwendung bei der Behandlung von Hauterkrankungen eingesetzt und beinhalten in der Regel keine weiteren Zusatzstoffe (WO 95/06459).

Zur optimalen Versorgung mit Nährstoffen müssen daher weitere Nahrungsergänzungsmittel, welche z.B. Frucht-, Gemüse- und Kräuterbestandteile sowie Vitamine, Mineralien und Enzyme beinhalten, eingenommen werden.

Nahrungsergänzungsmittel auf der Basis von Frucht-, Gemüse- und / oder Kräuterbestandteilen, die Vitamine, Mineralien und Enzyme beinhalten, zeichnen sich durch einen geringen Gehalt an Ballaststoffen aus und beinhalten nur geringe Mengen an Chlorophyll.

Chlorophyll ist strukturell dem Häm-Molekül ähnlich, welches bei Menschen und Tieren als Coenzym des Hämoglobins der roten Blutkörperchen Sauerstoffmoleküle bindet und für den Sauerstofftransport verantwortlich ist. Mit der Nahrung aufgenommenes Chlorophyll wird in Häm-Moleküle umgewandelt, wodurch die Hämoglobinbildung und der Sauerstofftransport gefördert werden. Da der grüne Teil der Gräser einen hohen Chlorophyllgehalt besitzt, werden Gräser als Nahrungsergänzungsmittel mit einer verbesserten Blutbildung und einem verbesserten Sauerstofftransport in Verbindung gebracht. Eine erhöhte Sauerstoffversorgung des Körpers, vor allem des Gehirns, fördert die Konzentration und wirkt gegen Antriebsschwäche.

Weiterhin besitzt Chlorophyll eine antioxidative Wirkung und ist daher in der Lage, schädliche Radikale zu binden und dadurch zu neutralisieren und so Zellen und Gewebe vor Schäden durch reaktive Radikale zu schützen.

Aufgrund der Eigenschaft des Chlorophylls Metall-Ionen zu komplexieren, wird Chlorophyll eine Bedeutung bei der Bindung und Ausscheidung von Schwermetall-lonen im Magen-Darmtrakt zugeschrieben.

Die in Gräsern in hohem Maße enthaltenen Ballaststoffe unterstützen die Verdauung, da sie im Darm, aufgrund ihrer Fähigkeit in große Mengen Wasser zu binden, aufquellen und damit Druck auf die Darmwände ausüben, wodurch die Peristaltik angeregt wird.

Darüber hinaus sorgt das Aufquellen der Ballaststoffe im Magen zu einer Verstärkung des Sättigungsgefühls, ohne dass es zu einem nennenswerten Anstieg des Blutzuckerspiegels kommt, da die Ballaststoffe im Verdauungstrakt zum Großteil nicht abgebaut werden. Ballaststoffreiche Ernährung ist daher insbesondere vorteilhaft für Diabetiker.

Weiterhin werden die in Gräsern enthaltenen Ballaststoffe mit einem cholesterinsenkenden Effekt in Verbindung gebracht, weshalb die Einnahme von Ballaststoffen die Anfälligkeit bzw. das Risiko für koronare Herzerkrankungen und somit beispielsweise das Risiko eines Herzinfarktes vermindert.

Ein geringer Teil der Ballaststoffe wird von der Darmflora abgebaut. Bei dieser Fermentation entstehen kurzkettige Fettsäuren wie z. B. Butyrat, welches der Entstehung von Krebs entgegen wirkt. Daher wird eine ballaststoffreiche Ernährung mit einer Reduktion des Darmkrebsrisikos in Verbindung gebracht.

Um eine optimale Versorgung mit Nährstoffen und eine optimale Krankheitsprävention bzw. Unterstützung der Genesung zu erreichen, ist daher bei einer Einnahme der in US 2006/0147563 A1 und WO 00/37087 A1 beschriebenen Nahrungsergänzungsmittel, welche keine Grasbestandteile beinhalten, die Einnahme weiterer Nahrungsergänzungsmittel, wie etwa die Einnahme von Ballaststoffen, nötig.

Weiterhin bestehen die bekannten Nahrungsergänzungsmittel aus einer festen Zusammensetzung von Bestandteilen, die auf den Ausgleich eines ganz spezifischen Defizits in der Nährstoffversorgung ausgerichtet ist. Ändert sich das Nährstoffbedürfnis, ist eine optimale Nährstoffversorgung mit den bekannten Nahrungsergänzungsmittein nicht mehr gewährleistet.

Da die Defizite in der Versorgung mit einzelnen Nährstoffen außerdem sehr individuell sind, ist für eine optimale Nährstoffversorgung eine variable Zusammenstellung des Nahrungsergänzungsmittels nach den individuellen Bedürfnissen erforderlich. Dies ist mit den bekannten Nahrungsergänzungsmittel, welche aus einer festen Zusammensetzung von Bestandteilen bestehen, nicht möglich.

Die Aufgabe der Erfindung ist es, ein verbessertes Nahrungsergänzungsmittel bereitzustellen, mit welchem eine bessere Versorgung des Organismus mit Nährstoffen und damit eine verbesserte Krankheitsprävention, eine Verbesserung des Allgemeinbefindens, sowie eine verbesserte Unterstützung der Genesung bzw. Heilung möglich ist. Weiterhin ist es die Aufgabe der Erfindung ein verbessertes Nahrungsergänzungsmittel bereitzustellen, welches eine einfache Anpassung an individuelle Versorgungsbedürfnisse ermöglicht. Eine weitere Aufgabe der Erfindung ist die Bereitstellung eines Herstellungsverfahrens für ein verbessertes Nahrungsergänzungsmittel sowie die Verwendung des Nahrungsergänzungsmittels zur Verbesserung des allgemeinen Wohlbefindens bzw. Gesundheitszustands.

Diese Aufgaben werden durch ein Nahrungsergänzungsmittel mit den Merkmalen gemäß Anspruch 1, einem Verfahren zur Herstellung eines Nahrungsergänzungsmittels mit den Merkmalen gemäß Anspruch 8 und einer Verwendung des Nahrungsergänzungsmittels mit den Merkmalen gemäß Anspruch 10 gelöst.

Zweckmäßige Weiterbildungen bzw. besondere Ausführungsformen sind in den unabhängigen Ansprüchen definiert.

Das erfindungsgemäße Nahrungsergänzungsmittel umfasst einen Gemüsebestandteil, einen Grasbestandteil, einen Fruchtbestandteil und ein Vitamin. Bevorzugt umfasst das Nahrungsergänzungsmittel mindestens einen weiteren Bestandteil, wobei der weitere Bestandteil ausgewählt ist aus der Gruppe bestehend aus einem Antioxidationsmittel, einem Coenzym, einem Mineral, einem Protein, einer Aminosäure, Zink-Citrat, Curcumin, L-Carnitin, Taurin, einem Kräuterbestandteil, einem Fischbestandteil und einem Nussbestandteil.

Gemüse im Sinne der Erfindung umfasst die Gesamtheit der Pflanze oder Teile der Pflanze (z. B. Wurzel, Stengel, Blatt, Blüte, Blüte im Zustand der Samenreife oder Samen). Weiterhin umfasst der Begriff des Gemüses die Pflanze oder Teile der Pflanze in allen ihren Entwicklungszuständen (z. B. Keimling oder Blüte im Zustand der Samenreife).

Bevorzugt ist der Gemüsebestandteil des Nahrungsergänzungsmittels ausgewählt aus der Gruppe bestehend aus Karotte, Kürbis, Pilz, Zucchini, Kartoffel, Aubergine, Spargel, Artischocke, Paprika, Salat, Gurke, Fenchel, Rote Bete, Radieschen, Schwarzwurzel, Sellerie, Tomate, Mangold, Kohl, Wirsing, Kohlrabi, Lauch, Spinat, Blumenkohl, Broccoli, Bohnen und Erbsen. Bevorzugt umfasst der Gemüsebestandteil ein oder mehrere Gemüse. Mehrere Gemüse im Sinne der Erfindung umfassen mehrere unterschiedliche Gemüse (z. B. Karotte oder Pilz) oder mehrere verschiedene Arten oder Sorten des gleichen Gemüses (z. B. bei Kartoffel beispielsweise die Sorten Agata oder Innovator). Besonders bevorzugt umfasst der Gemüsebestandteil zwei, drei, vier, fünf, sechs, sieben, acht, neun oder zehn Gemüse.

Die Pilze sind insbesondere ausgewählt aus der Gruppe bestehend aus Shütake-Pilz, Maitake-Pilz, Judasohr, Glänzender Lackporling, Igelstachelbart, Schopftintling, Eichhase, Steinpilz, Champignon, Austernpilz, Kräuterseitling, Maronenröhrling, Pfifferling, Stockschwämmchen, Trüffelpilz, Semmelstoppelpilz und Riesenbovist.

Gras im Sinne der Erfindung umfasst Pflanzenarten, welche der Ordnung der Poales angehören. Bevorzugt sind Grasarten, welche der Familie der Poaceae angehören. Zu der Familie der Poaceae zählen unter anderem die wichtigsten Getreidearten. Weiterhin bevorzugt sind Grasarten der Familien Cyperaceae und Juncaceae, zu denen die Papyrus-Pflanze und Binsen-Gewächse gehören.

Als Gras wird dabei die Gesamtheit sowie Teile (z. B. Wurzel, Stengel, Blatt, Blüte, Blüte im Zustand der Samenreife oder Samen) der Pflanze in all ihren Entwicklungsstadien (z. B. Keimling oder Blüte im Zustand der Samenreife) verstanden. Bevorzugt ist der Grasbestandteil ausgewählt aus der Gruppe bestehend aus Getreide, Gerste, Hirse, Hafer, Dinkel, Roggen, Mais, Weizen, Bambus, Zitronengras, Zuckerrohr und Reis. Bevorzugt umfasst der Grasbestandteil ein oder mehrere Gräser, besonders bevorzugt zwei, drei, vier, fünf, sechs, sieben, acht, neun oder zehn Gräser. Mehrere Gräser im Sinne der Erfindung umfassen mehrere unterschiedliche Gräser (z. B. Weizen oder Gerste) oder mehrere verschiedene Arten oder Sorten des gleichen Grases (z. B. bei Gerste beispielsweise die Sorten Birgit oder Brunhild).

Als Frucht im Sinne der Erfindung wird die Gesamtheit oder Teile (z. B. Wurzel, Stengel, Blatt, Blüte, Blüte im Zustand der Samenreife oder Samen) der Pflanze in all ihren Entwicklungsstadien (z. B. Keimling oder Blüte im Zustand der Samenreife) verstanden.

Bevorzugt ist der Fruchtbestandteil des Nahrungsergänzungsmittels ausgewählt aus der Gruppe bestehend aus Ananas, Johannisbeere, Wolfsbeere, Granatapfel, Apfel, Birne, Kiwi, Banane, Orange, Mandarine, Kirsche, Himbeere, Heidelbeere, Brombeere, Stachelbeere, Aprikose, Pfirsich, Pflaume, Kaki, Feige, Dattel, Erdbeere, Mango, Litschi, Melone, Traube, Papaya, Passionsfrucht und Pampelmuse. Bevorzugt umfasst der Fruchtbestandteil einen oder mehrere Früchte, besonders bevorzugt zwei, drei, vier, fünf, sechs, sieben, acht, neun oder zehn Früchte. Mehrere Früchte im Sinne der Erfindung umfassen mehrere unterschiedliche Früchte (z. B. Kiwi oder Birne) oder mehrere verschiedene Arten oder Sorten der gleichen Frucht (z. B. bei Apfel beispielsweise die Sorten Golden Delicious oder Jonagold).

Bevorzugt ist das Vitamin des Nahrungsergänzungsmittels ausgewählt aus der Gruppe bestehend aus Vitamin C, D-alpha-Tocopherol (Vitamin E), Vitamin B1 (Thiamin), Vitamin B2 (Riboflavin), Niacin (Vitamin B3), Pantothensäure (Vitamin B5), Vitamin B6 (Pyridoxin), Vitamin B12, Folsäure (Vitamin B9), Biotin (Vitamin H), Vitamin A, Vitamin D und Vitamin K. Bevorzugt umfasst das Nahrungsergänzungsmittel ein oder mehrere Vitamine, besonders bevorzugt zwei, drei, vier, fünf, sechs, sieben, acht, neun oder zehn Vitamine.

Unter Vitaminen im Sinne der Erfindung werden auch Derivate von Vitaminen verstanden. Die Vitaminderivate besitzen bevorzugt dieselbe oder eine verbesserte Wirkung als die korrespondierenden Vitamine. Die Vitaminderivate wirken bereits in der Verabreichungsform oder werden bevorzugt im Körper chemisch verändert und entfalten in der veränderten Form ihre Wirkung. Bevorzugte Vitaminderivate sind Oxidationsprodukte oder essbare Salze der Vitamine.

Beispielsweise sind bevorzugte Derivate von Vitamin C (Ascorbinsäure) die Dehydroascorbinsäure oder essbare Salze der Ascorbinsäure wie Calcium-, Natrium-, Magnesium-, Kalium- oder Zinkascorbat. Besonders bevorzugte Derivate von Vitamin C sind Calcium- oder Natriumascorbat. Ein weiterhin bevorzugtes Derivat von Vitamin C ist Ester-C.

Bevorzugt umfasst das Nahrungsergänzungsmittel weiterhin ein Coenzym. Coenzyme, zu denen auch Kofaktoren und prosthetische Gruppen zählen, sind in Proteine eingelagerte oder an Proteine kovalent oder nicht-kovalent gebundene Nichtprotein-Moleküle oder Metallionen. Zwischen den Begriffen Vitamin und Coenzym gibt es Überschneidungen, da einige Vitamine als Coenzyme fungieren (z. B. Biotin).

Bevorzugt enthält das Nahrungsergänzungsmittel ein oder mehrere Antioxidationsmittel. Antioxidationsmittel zeichnen sich bevorzugt dadurch aus, dass sie in der Lage sind, freie Radikale zu binden und dadurch zu neutralisieren. Dies ermöglicht, oxidative Schäden an Zellen und Geweben, durch freie Radikale verursacht, zu reduzieren bzw. zu verhindern.

Bevorzugt enthält das Nahrungsergänzungsmittel ein Antioxidationsmittel, welches ausgewählt ist aus der Gruppe bestehend aus oligomerem Procyanidin, Zitronensäure, Carotinoide, beta-Carotin, Lycopin, Flavonoide, Lecithin, Gallat, Milchsäure, Ascorbylpalmitat, Butylhydroxyanisol und Butylhydroxytoluol. Bevorzugt umfasst das Nahrungsergänzungsmittel ein oder mehrere Antioxidationsmittel, besonders bevorzugt zwei, drei, vier, fünf, sechs, sieben, acht, neun oder zehn Antioxidationsmittel.

In einer bevorzugten Ausführungsform wirken bestimmte Vitamine oder Vitaminderivate als Antioxidationsmittel. Bevorzugt wird Vitamin C oder D-alpha-Tocopherol (Vitamin E) als Antioxidationsmittel eingesetzt.

Bevorzugt umfasst das Nahrungsergänzungsmittel einen Kräuterbestandteil. Als Kräuter wird die Gesamtheit oder Teile (z. B. Wurzel, Stengel, Blatt, Blüte, Blüte im Zustand der Samenreife oder Samen) in allen Entwicklungsstadien der Pflanze (z. B. Keimling) bzw. ihrer Einzelteile (z. B. Blüte im Zustand der Samenreife) verstanden. Bevorzugt umfasst das Nahrungsergänzungsmittel einen Kräuterbestandteil, welcher ausgewählt ist aus der Gruppe bestehend aus Melisse, Enzian, Rosmarin, Hibiskus, Zwiebel, Knoblauch, Kresse, Oregano, Basilikum, Thymian, Petersilie, Koriander, Buchweizen, Bärlauch, Beifuß, Johanniskraut, Minze, Salbei, Kamille, Lavendel, Waldmeister, Zimt, Ginseng, Brennnessel, Baldrian, Ingwer, Hopfen, Hirtentäschel, Kümmel, Malve, Löwenzahn, Schafgarbe und Wegerich. Bevorzugt umfasst der Kräuterbestandteil ein oder mehrere Kräuter, besonders bevorzugt zwei, drei, vier, fünf, sechs, sieben, acht, neun oder zehn Kräuter. Mehrere Kräuter im Sinne der Erfindung umfassen mehrere unterschiedliche Kräuter (z. B. Melisse oder Rosmarin) oder mehrere verschiedene Arten oder Sorten der gleichen Kräuter (z. B. bei Hopfen beispielsweise die Sorten Nugget oder Smaragd).

In einer bevorzugten Ausführungsform umfasst das Nahrungsergänzungsmittel einen Fischbestandteil. Unter Fisch wird die Gesamtheit oder Teile (z. B. Fleisch, Organe, Knorpel oder Knochen) des Fisches in all seinen Entwicklungsstadien verstanden. Bevorzugt umfasst das Nahrungsergänzungsmittel einen Fischbestandteil, welcher ausgewählt ist aus der Gruppe bestehend aus Haifisch, Thunfisch, Lachs, Makrele, Hering, Kabeljau, Forelle, Dorsch, Scholle, Karpfen und Seelachs. Bevorzugt umfasst der Fischbestandteil einen oder mehrere Fische. Besonders bevorzugt umfasst der Fischbestandteil zwei, drei, vier, fünf, sechs, sieben, acht, neun oder zehn Fische. Mehrere Fische im Sinne der Erfindung umfassen mehrere unterschiedliche Fische (z. B. Haifisch oder Forelle) oder mehrere verschiedene Sorten oder Arten des gleichen Fischs (z. B. bei Forelle beispielsweise die Arten Bachforelle oder Regenbogenforelle).

Weiterhin umfasst das Nahrungsergänzungsmittel bevorzugt einen Nussbestandteil. Als Nuss im Sinne der Erfindung ist eine Pflanze zu verstehen, deren Blüte im Zustand der Samenreife in Form einer Nussfrucht ausgebildet ist. Eine Nuss umfasst dabei die Gesamtheit oder Teile (z. B. Wurzel, Stengel, Blatt, Blüte, Blüte im Zustand der Samenreife oder Samen) der Pflanze in all ihren Entwicklungsstadien (z. B. Keimling) bzw. in allen Entwicklungsstadien der Teile der Pflanze (z. B. Blüte im Zustand der Samenreife). Bevorzugt umfasst das Nahrungsergänzungsmittel einen Nussbestandteil, welcher ausgewählt ist aus der Gruppe bestehend aus Walnuss, Haselnuss, Paranuss, Buchecker, Kastanie, Mandel, Kokosnuss, Pistazie, Cashewnuss, Muskatnuss, Erdnuss und Pinienkern. Bevorzugt umfasst der Nussbestandteil ein oder mehrere Nüsse, besonders bevorzugt ein, zwei, drei, vier, fünf, sechs, sieben, acht, neun oder zehn Nüsse. Mehrere Nüsse im Sinne der Erfindung umfassen mehrere unterschiedliche Nüsse (z.B. Kastanie oder Haselnuss) oder mehrere verschiedene Sorten oder Arten der gleichen Nuss (z.B. bei Haselnuss beispielsweise die Arten gemeine Hasel oder Lambertshasel).

In einer bevorzugten Ausführungsform umfasst das Nahrungsergänzungsmittel ein Mineral. Bevorzugt umfasst das Mineral eine oder mehrere Verbindungen oder ein oder mehrere Salze umfassend ein Element ausgewählt aus der Gruppe bestehend aus Calcium, Phosphor, Magnesium, Selen, Zink, Eisen, Jod, Kobalt, Kupfer, Mangan, Natrium und Schwefel. Insbesondere umfasst das Mineral ein Mineral, welches ausgewählt ist aus der Gruppe bestehend aus Calciumcarbonat, Natriumselenat, Natriumhydrogencarbonat, Magnesiumcarbonat, Kaliumjodid, Calciumphosphat, Zinkoxid, Mangansulfat, Natriumchlorid, Titaniumdioxid und Kaliumchlorid. Bevorzugt umfasst das Nahrungsergänzungsmittel ein oder mehrere Mineralien. Besonders bevorzugt umfasst das Nahrungsergänzungsmittel zwei, drei, vier, fünf, sechs, sieben, acht, neun oder zehn Mineralien.

In einer bevorzugten Ausführungsform umfasst das Nahrungsergänzungsmittel ein Protein. Das Protein ist dabei bevorzugt aus Pflanzen oder Tieren extrahiert. Bevorzugt umfasst das Protein ein oder mehrere Proteine aus Tierprodukten (z. B. Milchprotein, Molkeprotein oder Eiprotein). In einer alternativen Ausführungsform ist das Protein dem Nahrungsergänzungsmittel in Form von Pflanzen- oder Tierbestandteilen beigefügt. In einer weiteren alternativen Ausführungsform wird das Protein aus Mikroorganismen extrahiert. Besonders bevorzugt wird das Protein dem Nahrungsergänzungsmittel in Form eines Extrakts aus Mikroorganismen beigefügt. Besonders bevorzugt ist das Protein ein Hefeextrakt. Weiterhin bevorzugt umfasst das Protein ein Proteinhydrolysat. Bevorzugt ist das Protein Pepton, Trypton oder Caseinhydrolysat.

Bevorzugt umfasst das Nahrungsergänzungsmittel ein Protein, welches ausgewählt ist aus der Gruppe bestehend aus Papain, Bromealin, Albumin, Collagen, Milchprotein, Molkeprotein und Eiprotein. Bevorzugt umfasst das Nahrungsergänzungsmittel ein oder mehrere Proteine, besonders bevorzugt zwei, drei, vier, fünf, sechs, sieben, acht, neun oder zehn Proteine.

Weiterhin umfasst das Nahrungsergänzungsmittel bevorzugt eine Aminosäure. Beispielsweise ist die Aminosäure ausgewählt aus der Gruppe bestehend aus L-Arginin, L-Glutamin, L-Alanin, L-Asparagin, L-Asparaginsäure, L-Cystein, L-Glutaminsäure, Glycin, L-Histidin, L-Isoleucin, L-Leucin, L-Lysin, L-Methionin, L-Phenylalanin, L-Prolin, L-Serin, L-Threonin, L-Tryptophan, L-Tyrosin, L-Valin, L-Zelinocystein und L-Pyrrolysin. Bevorzugt umfasst das Nahrungsergänzungsmittel eine oder mehrere Aminosäuren, bevorzugt zwei, drei, vier, fünf, sechs, sieben, acht, neun oder zehn Aminosäuren.

Weiterhin umfasst das Nahrungsergänzungsmittel Zink-Citrat, Curcumin, L-Carnitin und / oder Taurin.

Die einzelnen Bestandteile des Nahrungsergänzungsmittels (z.B. Gemüsebestandteil oder Vitamin) liegen bevorzugt in fester oder flüssiger Form vor. Besonders bevorzugt sind die Bestandteile beschaffen als Pulver, Flocken, Mehl, Kristalle, Granulat, wässrige Lösung, Öl und / oder Sirup. Besonders bevorzugt sind Kombinationen von Bestandteilen unterschiedlicher Form bzw. Beschaffenheit.

Bevorzugt liegen die Komponenten des Nahrungsergänzungsmittels (z.B. Gemüsebestandteil, Kräuterbestandteil, Fruchtbestandteil, Protein oder Aminosäure) als Extrakt vor bzw. liegen extrahiert vor. Unter einem Extrakt bzw. unter Extrahieren im Sinne der Erfindung wird verstanden, dass Inhaltsstoffe aus ihrer natürlichen Umgebung angereichert, aufgereinigt oder rein vorliegen.

Beispielsweise liegt das Vitamin, das Protein oder die Aminosäure des erfindungsgemäßen Nahrungsergänzungsmittels in gereinigter Form vor. In einer alternativen Ausführungsform ist das Vitamin aus einer Frucht, aus einem Gemüse, aus einem Fisch, aus Kräutern und / oder aus einer Nuss angereichert oder aufgereinigt. In einer weiteren alternativen Ausführungsform ist das Vitamin in einem Frucht-, Gemüse-, Kräuter-, Fisch- und / oder Nussbestandteil enthalten.

Alternativ ist das Protein dem Nahrungsergänzungsmittel in Form von Pflanzen- oder Tierextrakten beigefügt. Beispielsweise ist Bromealin und / oder Papain aus Papaya und / oder Ananas extrahiert.

L-Arginin und / oder L-Glutamin sind beispielsweise als Reisextrakt dem Nahrungsergänzungsmittel zugegeben.

Bevorzugt sind Vitamin, Antioxidationsmittel, Coenzym, Protein, Aminosäure, Zink-Citrat, Curcumin, L-Carnitin und / oder Taurin synthetisch oder mit gentechnischen Methoden hergestellt.

Bevorzugt umfasst das Nahrungsergänzungsmittel eine Grundkomponente, welche eine Vielzahl von Nährstoffen umfasst, die den Grundbedarf von Organismen an Nährstoffen deckt und eine variable Komponente, die zusätzliche Nährstoffe umfasst, die auf besondere Bedürfnisse eines Organismus abgestimmt sind.

Besonders bevorzugt umfasst das Nahrungsergänzungsmittel eine Grundkomponente und eine variable Komponente, wobei die Grundkomponente die Bestandteile Gemüse, Gras, Frucht, ein Vitamin und ein Oxidationsmittel umfasst und die variable Komponente mindestens einen weiteren Bestandteil umfasst ausgewählt aus der Gruppe bestehend aus Gemüse, Frucht, Kräuter, Pilz, Fisch, ein Mineral, ein Protein, eine Aminosäure, Zink-Citrat, Curcumin, L-Carnitin und Taurin. Beispielsweise umfasst die Grundkomponente einen Karottenbestandteil, einen Gerstengrasbestandteil, einen Ananasbestandteil, einen Johannisbeerbestandteil, ein Vitamin und ein Antioxidationsmittel, und die variable Komponente umfasst mindestens einen Bestandteil ausgewählt aus der Gruppe bestehend aus Kürbis, Melisse, Shiitake-Pilz, Enzian, Wolfsbeere, Rosmarin, Hibiskus, Granatapfel, Maitake-Pilz, Haifischknorpel, ein Mineral, ein Protein, eine Aminosäure, Zinkcitrat, Curcumin, L-Carnitin und Taurin.

Insbesondere bevorzugt umfasst das Nahrungsergänzungsmittel eine Grundkomponente umfassend einen Karottenbestandteil, einen Gerstengrasbestandteil, einen Ananasbestandteil, einen schwarze Johannisbeerbestandteil, Calcium-Ascorbat, D-alpha-Tocopherol, Vitamin B1, Vitamin B2, Niacin, Pantothensäure, Vitamin B6, Vitamin B12, Folsäure, Biotin und oligmeres Procyanidin und eine variable Komponente, umfassend einen oder mehrere Bestandteile ausgewählt aus der Gruppe bestehend aus Kürbis, Melisse, Shütake-Pilz, Enzian, Wolfsbeere, Rosmarin, Hibiskus, Granatapfel, Maitake-Pilz, Haifischknorpel, Calciumcarbonat, Natriumselenat, Papain, Bromealin, L-Arginin, L-Glutamin, Zink-Citrat, Curcumin, L-Carnitin und Taurin. Beispielsweise umfasst das Nahrungsergänzungsmittel eine variable Komponente, welche 1 - 20, bevorzugt 1 - 10, besonders bevorzugt 10 - 20, insbesondere bevorzugt 10-15, noch mehr bevorzugt 1 - 8, insbesondere bevorzugt 1 - 5, am meisten bevorzugt 1 - 3 Bestandteile umfasst, welche ausgewählt sind aus der Gruppe bestehend aus Kürbis, Melisse, Shütake-Pilz, Enzian, Wolfsbeere, Rosmarin, Hibiskus, Granatapfel, Maitake-Pilz, Haifischknorpel, Calciumcarbonat, Natriumselenat, Papain, Bromealin, L-Arginin, L-Glutamin, Zink-Citrat, Curcumin, L-Carnitin und Taurin. Bevorzugt umfasst die variable Komponente ein, zwei, drei, vier, fünf, sechs, sieben, acht, neun oder zehn Bestandteile ausgewählt aus der Gruppe bestehend aus Kürbis, Melisse, Shiitake-Pilz, Enzian, Wolfsbeere, Rosmarin, Hibiskus, Granatapfel, Maitake-Pilz, Haifischknorpel, Calciumcarbonat, Natriumselenat, Papain, Bromealin, L-Arginin, L-Glutamin, Zink-Citrat, Curcumin, L-Carnitin und Taurin.

Der Anteil des Gemüsebestandteils beträgt beispielsweise bezogen auf das Gewicht des Nahrungsergänzungsmittels insbesondere 12 - 30 %, der Anteil des Grasbestandteils insbesondere 5 - 15 %, der Anteil des Fruchtbestandteils insbesondere 15 - 35 %, der Anteil des Vitamins insbesondere 20 - 25 %, der Anteil des Antioxidationsmittels insbesondere 2 - 19 %, der Anteil des Kräuterbestandteils insbesondere 2 - 25 %, der Anteil des Fischbestandteils insbesondere 2 - 7 %, der Anteil des Minerals insbesondere 1 - 10 %, der Anteil des Proteins insbesondere 1 - 6 %, der Anteil der Aminosäure insbesondere 2 - 12 %, der Anteil des Zink-Citrats insbesondere 4 - 15 %, der Anteil des Curcumins insbesondere 1 - 8 %, der Anteil des L-Carnitins insbesondere 4 - 8 % und der Anteil des Taurins insbesondere 3 - 8 %.

Weiterhin richtet sich die Erfindung auf ein Verfahren zur Herstellung eines erfindungsgemäßen Nahrungsergänzungsmittels. Bevorzugt werden die Bestandteile des Nahrungsergänzungsmittels mit einem pharmazeutischen Träger versehen. Beispielsweise ist der pharmazeutische Träger ausgewählt aus der Gruppe bestehend aus Cellulose, wie Carboxymethylcellulose oder microcristalliner Cellulose, Alginat, Agarose, Alkohol, Wasser, Kochsalzlösung, Öl, Milchzucker und Paraffin. Das Verfahren zur Herstellung des Nahrungsergänzungsmittels umfasst die Zugabe von einem oder mehreren pharmazeutischen Trägern, die gleichzeitig und / oder nacheinander zugegeben werden. Bevorzugt werden durch Variation des Anteils des pharmazeutischen Trägers die Massenunterschiede von verschiedenen variablen Komponenten ausgeglichen.

Bevorzugt wird nach dem erfindungsgemäßen Verfahren das Nahrungsergänzungsmittel mit einem pharmazeutischen Träger versehen und in eine Darreichungsform gebracht, die vorzugsweise fest ist. Bevorzugte feste Darreichungsformen sind Tabletten, Pulver oder Kapseln, insbesondere Cellulosekapseln. In einer alternativen Ausführungsform ist die Darreichungsform flüssig, beispielsweise in Form von Tropfen, Saft oder Sirup. In einer weiteren Ausführungsform ist die Darreichungsform ein Feststoff, wie ein Pulver oder eine Tablette, welcher sich zum Auflösen in einer Flüssigkeit eignet.

Bevorzugt umfasst das Herstellungsverfahren des erfindungsgemäßen Nahrungsergänzungsmittels eine separate Herstellung der Grundkomponente und der variabler Komponente und eine anschließende Vereinigung der beiden Komponenten sowie die anschließende Zugabe des pharmazeutischen Trägers. In einer alternativen Ausführungsform wird der pharmazeutische Träger vor der Vereinigung der Grundkomponente und der variablen Komponente zur Grundkomponente und / oder zur variablen Komponente gegeben. Bevorzugt wird die Grundkomponente erstellt und zur Vereinigung mit den unterschiedlichen variablen Komponenten aufgeteilt. Alternativ werden die Bestandteile der Grundkomponente und der variablen Komponente zu Beginn des Herstellungsverfahrens vermengt.

Einseitige Ernährung und erhöhter Stress führen beispielsweise zu Defiziten in der Versorgung mit wichtigen Nährstoffen bzw. erhöhtem Verbrauch an Nährstoffen. Abhängig von der körperlichen Grundkonstitution eines Organismus treten Versorgungsdefizite auf. So führt beispielsweise eine ineffiziente Aufnahme und Verwertung bestimmter Bestandteile aus der Nahrung, welche individuell sehr unterschiedlich ausgeprägt ist, zu einem Versorgungsdefizit. Die so hervorgerufenen Defizite in der Versorgung mit wichtigen Nährstoffen haben beispielsweise Mangelerscheinungen und möglicherweise eine Einschränkung des Wohlbefindens zur Folge, die bis zum Auftreten von Krankheiten führen können.

Das erfindungsgemäße Nahrungsergänzungsmittel dient unter anderem zur Verbesserung des allgemeinen Gesundheitszustands von Mensch und Tier. Bevorzugt wird das Nahrungsergänzungsmittel zur Verbesserung der Immunabwehr, der Kreislauffunktion, der Venenfunktion, des Antriebs, der Aktivität, der Krankheitsanfälligkeit, der Nervenschwäche, der Sauerstoffversorgung, der Lungenfunktion, der Wundheilung, der Verdauung, der Blutqualität, des Knochen- und / oder Knorpelbaus, der Konzentration, des Blutzuckerspiegels, des Herzinfarktrisikos, des Sexualtriebs, der Psyche und / oder der Widerstandsfähigkeit gegen Krebs, sowie zur Verringerung der Kopfschmerzanfälligkeit, der Suchtanfälligkeit und / oder der Neigung zu Kropferkrankungen verwendet.

Es ist bekannt, dass bei Personen, die unter einem bestimmten Sternzeichen geboren sind, bestimmte Krankheiten besonders häufig auftreten bzw. spezifische Schwachpunkte des allgemeinen Gesundheitszustands vorliegen. Bei Personen, die unter dem Sternzeichen Steinbock geboren sind, treten insbesondere Nieren, Darm-, Haut- und Knochenbeschwerden sowie Stoffwechselentgleisungen auf. Personen, die unter dem Sternzeichen Stier geboren wurden, leiden häufig an einem schwachen Knochenbau sowie Kopf, Magen-, Darm- oder Halsleiden und neigen zu Kropferkrankungen. Personen, die unter dem Sternzeichen Zwilling geboren sind, haben häufig Nerven-, Darm- und Leberleiden und Erkältungen der Brustorgane sowie Suchtdispositionen. Weibliche Personen, die unter dem Sternzeichen Zwilling geboren sind, leiden gehäuft an Hysterie. Personen, die unter dem Sternzeichen Krebs geboren sind, neigen zu Magenleiden, Darmstörungen, psychische Krankheiten und Blutkrankheiten und leiden häufig unter seelischen Belastungen. Personen, die unter dem Sternzeichen Skorpion geboren sind, neigen zu Infektionen, Nieren- und Enddarmerkrankungen, Sexualleiden und Bluterkrankungen. Bei Personen, die unter dem Sternzeichen Waage geboren sind, treten vermehrt Nieren-, Kopf-, Haut- und Gefäßleiden sowie Krankheiten des Bewegungsapparates und Durchblutungsstörungen auf. Personen, die unter dem Sternzeichen Jungfrau geboren sind, leiden häufig unter Leber-, Milz- und Darmbeschwerden sowie unter Störungen des Knochenbaus und der Verdauungssäfte. Personen, die unter dem Sternzeichen Löwe geboren sind, neigen zu Störungen der Durchblutung, Infektionen und Magen- und Darmbeschwerden. Personen, die unter dem Sternzeichen Schütze geboren sind, leiden häufig unter Nervenerkrankungen, Muskelverspannungen im Schulter-Hals-Bereich, Kopfschmerz, Ischias und Gliederlähmungen. Personen, die unter dem Sternzeichen Widder geboren sind, neigen zu Knochenproblemen und Knorpelschäden sowie Nervenleiden, Darmerkrankungen und Kreislaufstörungen. Personen, die unter dem Sternzeichen Wassermann geboren sind, haben häufiger Wadenvenenleiden, Nerven-, Herz- und Lungenerkrankungen sowie Kreislaufprobleme und Durchblutungsstörungen. Personen, die unter dem Sternzeichen Fische geboren sind, neigen zu Fußleiden, Herz-, Darm- und Atmungsbeschwerden und Erkrankungen der Bronchien.

In einer bevorzugten Ausführungsform des Nahrungsergänzungsmittels, umfasst das Nahrungsergänzungsmittel eine Grundkomponente und eine variable Komponente, wobei die variable Komponente auf spezifische Gesundheitsrisiken bzw. Schwachpunkte im allgemeinen Gesundheitszustand von Lebewesen abgestimmt ist. Solche Schwachpunkte bzw. Neigungen zu Erkrankungen korrelieren beispielsweise mit dem Sternzeichen eines Lebewesens so dass die variable Komponente bevorzugt auf das Sternzeichen, unter dem eine Person geboren ist, abgestimmt ist.

### Beispiele

### Beispiel 1

Nahrungsergänzungsmittel mit einer Grundkomponente und einer oder mehrerer variabler Komponenten in Form einer Cellulosekapsel mit microcristalliner Cellulose als pharmazeutischer Träger (2-40 mg pro Kapsel):

**Grundkomponente:**

| **Inhaltsstoffe** | **Kapselinhalt in mg** |
|---|---|
| Karottenpulver | 30,0 - 80,0 |
| Gerstengraspulver | 20,0 - 70 |
| Ananaspulver | 20,0 - 70 |
| Schwarze Johannisbeerpulver | 20,0 - 70 |
| Calciumascorbat (Vitamin Ester C) | 50,0 - 110,0 |
| D-alpha-Tocopherol | 5,0 - 15,0 |
| Oligmere Procyanidine (OPC) | 10,0 - 25,0 |
| Vitamin B1 | 0,6 - 2,2 |
| Vitamin B2 | 0,8 - 2,4 |
| Niacin (Vitamin B3) | 14,0 - 22,0 |
| Pantothensäure (Vitamin B5) | 2,0 - 10,0 |
| Vitamin B6 | 1,0 - 3,0 |
| Vitamin B12 (0,1 %) | 0,2 - 1,8 |
| Folsäure (Vitamin B9) | 0,1 - 0,3 |
| Biotin (Vitamin H) | 0,05 - 0,2 |

**Variable Komponente:**

| **Inhaltsstoffe** | **Kapselinhalt in mg** |
|---|---|
| Kürbiskerne gemahlen | 15,0 - 35,0 |
| Curcumin | 20,0 - 40,0 |
| Melisse | 30,0 - 50,0 |

oder

| **Inhaltsstoffe** | **Kapselinhalt in mg** |
|---|---|
| Shiitake-Pilzextrakt | 15,0 - 35,0 |
| L-Carnitin | 20,0 - 40,0 |
| Enzianextrakt | 30,0 - 50,0 |

oder

| **Inhaltsstoffe** | **Kapselinhalt in mg** |
|---|---|
| Wolfsbeere | 20,0 - 40,0 |
| Granatapfelextrakt | 10,0 - 30,0 |
| Melisse | 35,0 - 55,0 |

oder

| **Inhaltsstoffe** | **Kapselinhalt in mg** |
|---|---|
| Shiitake-Pilzextrakt | 15,0 - 35,0 |
| Maitake-Pilzextrakt | 15,0 - 35,0 |
| Melisse | 30,0 - 50,0 |

oder

| **Inhaltsstoffe** | **Kapselinhalt in mg** |
|---|---|
| Calciumcarbonat | 35,0 - 55,0 |
| Granatapfel-Extrakt | 5,0 - 25,0 |
| Haifischknorpelpulver | 15,0 - 35,0 |

oder

| **Inhaltsstoffe** | **Kapselinhalt in mg** |
|---|---|
| Zink-Citrat - 3 Hydrat (32 % Zn) | 40,0 - 60,0 |
| Natriumselenat (0,1 % Selen) | 1,0 - 20,0 |
| Papain | 10,0 - 30,0 |

oder

| **Inhaltsstoffe** | **Kapselinhalt in mg** |
|---|---|
| Papain | 10,0 - 30,0 |
| Bromealin (2000 GDU) | 5,0 - 25,0 |
| Rosmarinblattpulver | 45,0 - 65,0 |

oder

| **Inhaltsstoffe** | **Kapselinhalt in mg** |
|---|---|
| L-Arginin | 35,0 - 55,0 |
| Granatapfel-Extrakt | 10,0 - 30,0 |
| Melisse | 15,0 - 35,0 |

oder

| **Inhaltsstoffe** | **Kapselinhalt in mg** |
|---|---|
| Granatapfel-Extrakt | 40,0 - 60,0 |
| Taurin | 10,0 - 30,0 |
| L-Glutamin | 10,0 - 30,0 |

oder

| **Inhaltsstoffe** | **Kapselinhalt in mg** |
|---|---|
| Zink-Citrat - 3 Hydrat (32 % Zn) | 40,0 - 60,0 |
| Curcumin | 1,0 - 20,0 |
| Hibiskus-Anthozyane | 10,0 - 30,0 |

oder

| **Inhaltsstoffe** | **Kapselinhalt in mg** |
|---|---|
| Zink-Citrat - 3 Hydrat (32 % Zn) | 15,0 - 35,0 |
| Natriumselenat (0,1 % Selen) | 1,0 - 20,0 |
| Maiitake-Pilzextrakt | 30,0 - 50,0 |

oder

| **Inhaltsstoffe** | **Kapselinhalt in mg** |
|---|---|
| Calciumcarbonat | 30,0 - 50,0 |
| Bromealin | 1,0 - 20,0 |
| Taurin | 20,0 - 30,0 |

### Beispiel 2

Nahrungsergänzungsmittel mit einer Grundkomponente, einer oder mehrerer variabler Komponenten und microcristalliner Cellulose als pharmazeutischer Träger in Form einer Cellulosekapsel (95 mg pro Kapsel):

**Grundkomponente:**

| **Inhaltsstoffe** | **Kapselinhalt in mg** |
|---|---|
| Broccolipulver | 50,0 |
| Weizengraspulver | 40,0 |
| Organenpulver | 40,0 |
| Himbeerpulver | 40,0 |
| Calciumascorbat (Vitamin Ester C) | 80,0 |
| D-alpha-Tocopherol | 10,0 |
| Oligmere Procyanidine (OPC) | 15,0 |
| Vitamin B1 | 1,4 |
| Vitamin B2 | 1,6 |
| Niacin (Vitamin B3) | 18,0 |
| Pantothensäure (Vitamin B5) | 6,0 |
| Vitamin B6 | 2,0 |
| Vitamin B12 (0,1 %) | 1,0 |
| Folsäure (Vitamin B9) | 0,2 |
| Biotin (Vitamin H) | 0,15 |

### Variable Komponente und microcristalline Cellulose:

**Variable Komponente:**

| **Inhaltsstoffe** | **Kapselinhalt in mg** |
|---|---|
| Kürbiskerne gemahlen | 25,0 |
| Curcumin | 30,0 |
| Melisse | 40,0 |

| | |
|---|---|
| microcristalline Cellulose: 5 mg, | |

oder

**variable Komponente:**

| **Inhaltsstoffe** | **Kapselinhalt in mg** |
|---|---|
| Shiitake-Pilzextrakt | 25,0 |
| L-Carnitin | 30,0 |
| Enzianextrakt | 40,0 |

| | |
|---|---|
| microcristalline Cellulose: 5 mg, | |

oder

**variable Komponente:**

| **Inhaltsstoffe** | **Kapselinhalt in mg** |
|---|---|
| Wolfsbeere | 30,0 |
| Granatapfelextrakt | 20,0 |
| Melisse | 45,0 |

| | |
|---|---|
| microcristalline Cellulose: 5 mg, | |

oder

**variable Komponente:**

| **Inhaltsstoffe** | **Kapselinhalt in mg** |
|---|---|
| Shütake-Pilzextrakt | 25,0 |
| Maitake-Pilzextrakt | 25,0 |
| Melisse | 40,0 |

| | |
|---|---|
| microcristalline Cellulose: 10 mg, | |

oder

**variable Komponente:**

| **Inhaltsstoffe** | **Kapselinhalt in mg** |
|---|---|
| Calciumcarbonat | 45,0 |
| Granatapfel-Extrakt | 15,0 |
| Haifischknorpelpulver | 25,0 |

| | |
|---|---|
| microcristalline Cellulose: 15 mg, | |

oder

**variable Komponente:**

| **Inhaltsstoffe** | **Kapselinhalt in mg** |
|---|---|
| Zink-Citrat - 3 Hydrat (32 % Zn) | 50,0 |
| Natriumselenat (0,1 % Selen) | 10,0 |
| Papain | 20,0 |

| | |
|---|---|
| microcristalline Cellulose: 20 mg, | |

oder

**variable Komponente:**

| **Inhaltsstoffe** | **Kapselinhalt in mg** |
|---|---|
| Papain | 20,0 |
| Bromealin (2000 GDU) | 15,0 |
| Rosmarinblattpulver | 55,0 |

| | |
|---|---|
| microcristalline Cellulose: 10 mg, | |

oder

**variable Komponente:**

| **Inhaltsstoffe** | **Kapselinhalt in mg** |
|---|---|
| L-Arginin | 45,0 |
| Granatapfel-Extrakt | 20,0 |
| Melisse | 25,0 |

| | |
|---|---|
| microcristalline Cellulose: 10 mg, | |

oder

**variable Komponente:**

| **Inhaltsstoffe** | **Kapselinhalt in mg** |
|---|---|
| Granatapfel-Extrakt | 50,0 |
| Taurin | 20,0 |
| L-Glutamin | 20,0 |

| | |
|---|---|
| microcristalline Cellulose: 10 mg, | |

oder

**variable Komponente:**

| **Inhaltsstoffe** | **Kapselinhalt in mg** |
|---|---|
| Zink-Citrat - 3 Hydrat (32 % Zn) | 50,0 |
| Curcumin | 10,0 |
| Hibiskus-Anthozyane | 20,0 |

| | |
|---|---|
| microcristalline Cellulose: 20 mg, | |

oder

**variable Komponente:**

| **Inhaltsstoffe** | **Kapselinhalt in mg** |
|---|---|
| Zink-Citrat - 3 Hydrat (32 % Zn) | 25,0 |
| Natriumselenat (0,1 % Selen) | 10,0 |
| Maütake-Pilzextrakt | 40,0 |

| | |
|---|---|
| microcristalline Cellulose: 25 mg, | |

oder

**variable Komponente:**

| **Inhaltsstoffe** | **Kapselinhalt in mg** |
|---|---|
| Calciumcarbonat | 40,0 |
| Bromealin | 10,0 |
| Taurin | 30,0 |

| | |
|---|---|
| microcristalline Cellulose: 20 mg. | |

### Beispiel 3

Nahrungsergänzungsmittel mit einer Grundkomponente, einer oder mehrerer variabler Komponenten und microcristalliner Cellulose als pharmazeutischer Träger in Form einer Cellulosekapsel (95 mg pro Kapsel):

**Grundkomponente:**

| **Inhaltsstoffe** | **Kapselinhalt in mg** |
|---|---|
| Broccolipulver | 45,0 |
| Roggenpulver | 50,0 |
| Apfelpulver | 30,0 |
| Traubenpulver | 20,0 |
| Vitamin C | 80,0 |
| D-alpha-Tocopherol | 10,0 |
| beta-Carotin | 20,0 |
| Vitamin B1 | 1,6 |
| Vitamin B2 | 1,8 |
| Niacin (Vitamin B3) | 16,0 |
| Pantothensäure (Vitamin B5) | 8,0 |
| Vitamin B6 | 3,0 |
| Vitamin B12 (0,1 %) | 2,0 |
| Folsäure (Vitamin B9) | 0,5 |
| Biotin (Vitamin H) | 0,2 |
| Vitamin D | 2,0 |
| Vitamin K | 1,2 |
| Vitamin A | 0,5 |

### Variable Komponente und microcristalline Cellulose:

**Variable Komponente:**

| **Inhaltsstoffe** | **Kapselinhalt in mg** |
|---|---|
| Wolfsbeere | 50,0 |
| Curcumin | 40,0 |

| | |
|---|---|
| microcristalline Cellulose: 5 mg, | |

oder

**variable Komponente:**

| **Inhaltsstoffe** | **Kapselinhalt in mg** |
|---|---|
| Shiitake-Pilzextrakt | 25,0 |
| L-Carnitin | 25,0 |
| Enzianextrakt | 35,0 |
| Maitake-Pilzextrakt | 20,0 |

| | |
|---|---|
| microcristalline Cellulose: 8 mg, | |

oder

**variable Komponente:**

| **Inhaltsstoffe** | **Kapselinhalt in mg** |
|---|---|
| Natriumselenat (0,1 % Selen) | 15,0 |
| Granatapfelextrakt | 30,0 |
| Melisse | 40,0 |

| | |
|---|---|
| microcristalline Cellulose: 8 mg, | |

oder

**variable Komponente:**

| **Inhaltsstoffe** | **Kapselinhalt in mg** |
|---|---|
| Shütake-Pilzextrakt | 20,0 |
| Maitake-Pilzextrakt | 25,0 |
| Melisse | 35,0 |
| Granatapfel-Extrakt | 10,0 |
| Haifischknorpelpulver | 30,0 |

| | |
|---|---|
| microcristalline Cellulose: 20 mg, | |

oder

**variable Komponente:**

| **Inhaltsstoffe** | **Kapselinhalt in mg** |
|---|---|
| Granatapfel-Extrakt | 40,0 |
| Natriumselenat (0,1 % Selen) | 15,0 |
| Bromealin (2000 GDU) | 5,0 |

| | |
|---|---|
| microcristalline Cellulose: 15 mg, | |

oder

**variable Komponente:**

| **Inhaltsstoffe** | **Kapselinhalt in mg** |
|---|---|
| Zink-Citrat - 3 Hydrat (32 % Zn) | 40,0 |
| Natriumselenat (0,1 % Selen) | 15,0 |
| Papain | 15,0 |

| | |
|---|---|
| microcristalline Cellulose: 15 mg, | |

oder

**variable Komponente:**

| **Inhaltsstoffe** | **Kapselinhalt in mg** |
|---|---|
| Papain | 25,0 |
| Rosmarinblattpulver | 45,0 |
| Melisse | 35,0 |
| Hibiskus-Anthozyane | 20,0 |

| | |
|---|---|
| microcristalline Cellulose: 20 mg, | |

oder

**variable Komponente:**

| **Inhaltsstoffe** | **Kapselinhalt in mg** |
|---|---|
| L-Arginin | 40,0 |
| Granatapfel-Extrakt | 15,0 |
| Melisse | 20,0 |
| Zink-Citrat - 3 Hydrat (32 % Zn) | 45,0 |

| | |
|---|---|
| microcristalline Cellulose: 15 mg, | |

oder

**variable Komponente:**

| **Inhaltsstoffe** | **Kapselinhalt in mg** |
|---|---|
| Granatapfel-Extrakt | 40,0 |
| Taurin | 25,0 |
| L-Glutamin | 25,0 |

| | |
|---|---|
| microcristalline Cellulose: 15 mg, | |

oder

**variable Komponente:**

| **Inhaltsstoffe** | **Kapselinhalt in mg** |
|---|---|
| Zink-Citrat - 3 Hydrat (32 % Zn) | 45,0 |
| Curcumin | 15,0 |
| Hibiskus-Anthozyane | 25,0 |

| | |
|---|---|
| microcristalline Cellulose: 15 mg, | |

oder

**variable Komponente:**

| **Inhaltsstoffe** | **Kapselinhalt in mg** |
|---|---|
| Zink-Citrat - 3 Hydrat (32 % Zn) | 20,0 |
| Natriumselenat (0,1 % Selen) | 15,0 |
| Calciumcarbonat | 30,0 |

| | |
|---|---|
| microcristalline Cellulose: 20 mg, | |

oder

**variable Komponente:**

| **Inhaltsstoffe** | **Kapselinhalt in mg** |
|---|---|
| Calciumcarbonat | 10,0 |
| Bromealin | 20,0 |
| Taurin | 35,0 |
| Maiitake-Pilzextrakt | 40,0 |

| | |
|---|---|
| microcristalline Cellulose: 25 mg. | |

### Beispiel 4

Nahrungsergänzungsmittel mit einer Grundkomponente und einer oder mehrerer variabler Komponenten in Form einer Cellulosekapsel mit Carboxymethylcellulose als pharmazeutischer Träger:

**Grundkomponente:**

| **Inhaltsstoffe** | **Kapselinhalt in mg** |
|---|---|
| Broccolipulver | 45,0 |
| Roggenpulver | 50,0 |
| Apfelpulver | 30,0 |
| Haselnusspulver | 20,0 |
| Lycopin | 25,0 |
| Vitamin C | 80,0 |
| Vitamin B1 | 1,6 |
| Vitamin B2 | 1,8 |
| Vitamin B6 | 3,0 |
| Biotin (Vitamin H) | 0,2 |
| Vitamin D | 2,0 |
| Vitamin K | 1,2 |
| Vitamin A | 0,5 |

### Variable Komponente und microcristalline Cellulose:

**Variable Komponente:**

| **Inhaltsstoffe** | **Kapselinhalt in mg** |
|---|---|
| Buchweizen | 45,0 |
| Curcumin | 40,0 |

| | |
|---|---|
| Carboxymethylcellulose: 5 mg, | |

oder

**variable Komponente:**

| **Inhaltsstoffe** | **Kapselinhalt in mg** |
|---|---|
| Steinpilz-Extrakt | 15,0 |
| L-Carnitin | 20,0 |
| Hirtentäschel | 50,0 |

| | |
|---|---|
| Carboxymethylcellulose: 10 mg, | |

oder

**variable Komponente:**

| **Inhaltsstoffe** | **Kapselinhalt in mg** |
|---|---|
| Kaliumjodid | 20,0 |
| Granatapfelextrakt | 35,0 |
| Melisse | 40,0 |

| | |
|---|---|
| Carboxymethylcellulose: 10 mg, | |

oder

**variable Komponente:**

| **Inhaltsstoffe** | **Kapselinhalt in mg** |
|---|---|
| Minz-Extrakt | 40,0 |
| Natriumselenat (0,1 % Selen) | 15,0 |
| L-Glutamin | 5,0 |

| | |
|---|---|
| Carboxymethylcellulose: 20 mg, | |

oder

**variable Komponente:**

| **Inhaltsstoffe** | **Kapselinhalt in mg** |
|---|---|
| Zink-Citrat - 3 Hydrat (32 % Zn) | 30,0 |
| Natriumselenat (0,1 % Selen) | 15,0 |
| Papain | 15,0 |

| | |
|---|---|
| Carboxymethylcellulose: 10 mg, | |

oder

**variable Komponente:**

| **Inhaltsstoffe** | **Kapselinhalt in mg** |
|---|---|
| Wegerich-Extrakt | 25,0 |
| Rosmarinblattpulver | 25,0 |
| Melisse | 35,0 |
| Hibiskus-Anthozyane | 20,0 |

| | |
|---|---|
| Carboxymethylcellulose: 15 mg, | |

oder

**variable Komponente:**

| **Inhaltsstoffe** | **Kapselinhalt in mg** |
|---|---|
| Bärlauch | 40,0 |
| Granatapfel-Extrakt | 15,0 |
| Melisse | 20,0 |
| Zink-Citrat - 3 Hydrat (32 % Zn) | 10,0 |

| | |
|---|---|
| Carboxymethylcellulose: 10 mg, | |

oder

**variable Komponente:**

| **Inhaltsstoffe** | **Kapselinhalt in mg** |
|---|---|
| Pepton | 40,0 |
| Taurin | 25,0 |
| L-Glutamin | 15,0 |

| | |
|---|---|
| Carboxymethylcellulose: 10 mg, | |

oder

**variable Komponente:**

| **Inhaltsstoffe** | **Kapselinhalt in mg** |
|---|---|
| Oregano | 35,0 |
| Curcumin | 15,0 |
| Eiprotein | 35,0 |

| | |
|---|---|
| Carboxymethylcellulose: 10 mg, | |

oder

**variable Komponente:**

| **Inhaltsstoffe** | **Kapselinhalt in mg** |
|---|---|
| Zink-Citrat - 3 Hydrat (32 % Zn) | 15,0 |
| Zimt | 25,0 |
| L-Alanin | 10,0 |

| | |
|---|---|
| Carboxymethylcellulose: 10 mg, | |

oder

**variable Komponente:**

| **Inhaltsstoffe** | **Kapselinhalt in mg** |
|---|---|
| Calciumcarbonat | 10,0 |
| Waldmeister | 30,0 |
| Taurin | 25,0 |
| Maütake-Pilzextrakt | 40,0 |

| | |
|---|---|
| Carboxymethylcellulose: 20 mg, | |

oder

**variable Komponente:**

| **Inhaltsstoffe** | **Kapselinhalt in mg** |
|---|---|
| Shiitake-Pilzextrakt | 25,0 |
| L-Carnitin | 15,0 |
| Magnesiumcarbonat | 20,0 |
| Rosmarinxtrakt | 40,0 |

| | |
|---|---|
| Carboxymethylcellulose: 8 mg, | |

oder

**variable Komponente:**

| **Inhaltsstoffe** | **Kapselinhalt in mg** |
|---|---|
| Shütake-Pilzextrakt | 50,0 |
| Kamille-Extrakt | 60,0 |
| Calciumcarbonat | 20,0 |

| | |
|---|---|
| Carboxymethylcellulose: 10 mg, | |

oder

**variable Komponente:**

| **Inhaltsstoffe** | **Kapselinhalt in mg** |
|---|---|
| Hefe-Extrakt | 87,0 |

| | |
|---|---|
| Carboxymethylcellulose: 10 mg, | |

oder

**variable Komponente:**

| **Inhaltsstoffe** | **Kapselinhalt in mg** |
|---|---|
| Molkeprotein | 87,0 |

| | |
|---|---|
| Carboxymethylcellulose: 10 mg. | |

### Beispiel 5

Nahrungsergänzungsmittel mit einer Grundkomponente und einer oder mehrerer variabler Komponenten in Form einer Tablette mit Carboxymethylcellulose als pharmazeutischer Träger:

**Grundkomponente:**

| **Inhaltsstoffe** | **Tabletteninhalt in mg** |
|---|---|
| Fenchel-Extrakt | 45,0 |
| Maispulver | 50,0 |
| Erdbeerpulver | 30,0 |
| oligomeres Procyanidin | 25,0 |
| Vitamin C | 80,0 |
| Niacin | 1,6 |
| Pantothensäure | 1,8 |
| Vitamin B6 | 3,0 |
| Biotin (Vitamin H) | 0,2 |
| D-alpha-Tocophereol | 2,0 |
| Vitamin B12 | 1,2 |

### Variable Komponente und microcristalline Cellulose:

**Variable Komponente:**

| **Inhaltsstoffe** | **Tabletteninhalt in mg** |
|---|---|
| Salbei | 45,0 |
| Hopfen | 40,0 |
| Papain | 5,0 |

| | |
|---|---|
| Carboxymethylcellulose: 10 mg, | |

oder

**variable Komponente:**

| **Inhaltsstoffe** | **Tabletteninhalt in mg** |
|---|---|
| Steinpilz-Extrakt | 20,0 |
| Mangansulfat | 5,0 |
| Johanniskraut | 40,0 |
| Minz-Extrakt | 40,0 |

| | |
|---|---|
| Carboxymethylcellulose: 10 mg, | |

oder

**variable Komponente:**

| **Inhaltsstoffe** | **Tabletteninhalt in mg** |
|---|---|
| Calciumcarbonat | 10,0 |
| Rosmarin | 45,0 |
| Pepton | 40,0 |

| | |
|---|---|
| Carboxymethylcellulose: 10 mg, | |

oder

**variable Komponente:**

| **Inhaltsstoffe** | **Tabletteninhalt in mg** |
|---|---|
| L-Glutamin | 5,0 |
| Natriumselenat (0,1 % Selen) | 15,0 |
| Bärlauch | 40,0 |

| | |
|---|---|
| Carboxymethylcellulose: 15 mg, | |

oder

**variable Komponente:**

| **Inhaltsstoffe** | **Tabletteninhalt in mg** |
|---|---|
| Zink-Citrat - 3 Hydrat (32 % Zn) | 20,0 |
| Granatapfelextrakt | 35,0 |
| Steinpilz-Extrakt | 25,0 |

| | |
|---|---|
| Carboxymethylcellulose: 10 mg, | |

oder

**variable Komponente:**

| **Inhaltsstoffe** | **Tabletteninhalt in mg** |
|---|---|
| Rosmarinblattpulver | 20,0 |
| Melisse | 30,0 |
| Hibiskus-Anthozyane | 20,0 |
| Zink-Citrat - 3 Hydrat (32 % Zn) | 10,0 |

| | |
|---|---|
| Carboxymethylcellulose: 15 mg, | |

oder

**variable Komponente:**

| **Inhaltsstoffe** | **Tabletteninhalt in mg** |
|---|---|
| Granatapfel-Extrakt | 20,0 |
| Melisse | 20,0 |
| Zink-Citrat - 3 Hydrat (32 % Zn) | 10,0 |

| | |
|---|---|
| Carboxymethylcellulose: 10 mg, | |

oder

**variable Komponente:**

| **Inhaltsstoffe** | **Tabletteninhalt in mg** |
|---|---|
| Taurin | 15,0 |
| L-Glutamin | 10,0 |
| Curcumin | 15,0 |

| | |
|---|---|
| Carboxymethylcellulose: 10 mg, | |

oder

**variable Komponente:**

| **Inhaltsstoffe** | **Tabletteninhalt in mg** |
|---|---|
| Oregano | 35,0 |
| Eiprotein | 35,0 |
| Wegerich-Extrakt | 25,0 |

| | |
|---|---|
| Carboxymethylcellulose: 10 mg. | |

### Beispiel 6

Nahrungsergänzungsmittel mit einer Grundkomponente, einer oder mehrerer variabler Komponenten und microcristalliner Cellulose als pharmazeutischer Träger in Form einer Cellulosekapsel (95 mg pro Kapsel):

**Grundkomponente:**

| **Inhaltsstoffe** | **Kapselinhalt in mg** |
|---|---|
| Karottenpulver | 50,0 |
| Gerstengraspulver | 40,0 |
| Ananaspulver | 40,0 |
| Schwarze Johannisbeerpulver | 40,0 |
| Calciumascorbat (Vitamin Ester C) | 80,0 |
| D-alpha-Tocopherol | 10,0 |
| Oligmere Procyanidine (OPC) | 15,0 |
| Vitamin B1 | 1,4 |
| Vitamin B2 | 1,6 |
| Niacin (Vitamin B3) | 18,0 |
| Pantothensäure (Vitamin B5) | 6,0 |
| Vitamin B6 | 2,0 |
| Vitamin B12 (0,1 %) | 1,0 |
| Folsäure (Vitamin B9) | 0,2 |
| Biotin (Vitamin H) | 0,15 |

### Variable Komponente und microcristalline Cellulose:

**Variable Komponente:**

| **Inhaltsstoffe** | **Kapselinhalt in mg** |
|---|---|
| Kürbiskerne gemahlen | 25,0 |
| Curcumin | 30,0 |
| Melisse | 40,0 |

| | |
|---|---|
| Microcristalline Cellulose: 5 mg | |

Diese Zusammensetzung ist bevorzugt für Personen geeignet, welche unter dem Sternzeichen Krebs geboren sind,
oder

**variable Komponente:**

| **Inhaltsstoffe** | **Kapselinhalt in mg** |
|---|---|
| Shütake-Pilzextrakt | 25,0 |
| L-Carnitin | 30,0 |
| Enzianextrakt | 40,0 |

| | |
|---|---|
| Microcristalline Cellulose: 5 mg | |

Dieses Nahrungsergänzungsmittel eignet sich bevorzugt für Personen, die unter dem Sternzeichen Schütze geboren sind,
oder

**variable Komponente:**

| **Inhaltsstoffe** | **Kapselinhalt in mg** |
|---|---|
| Wolfsbeere | 30,0 |
| Granatapfelextrakt | 20,0 |
| Melisse | 45,0 |

| | |
|---|---|
| Microcristalline Cellulose: 5 mg | |

Dieses Nahrungsergänzungsmittel eignet sich bevorzugt für Personen, die unter dem Sternzeichen Skorpion geboren sind,
oder

**variable Komponente:**

| **Inhaltsstoffe** | **Kapselinhalt in mg** |
|---|---|
| Shütake-Pilzextrakt | 25,0 |
| Maitake-Pilzextrakt | 25,0 |
| Melisse | 40,0 |

| | |
|---|---|
| Microcristalline Cellulose: 10 mg | |

Dieses Nahrungsergänzungsmittel eignet sich bevorzugt für Personen, die unter dem Sternzeichen Löwe geboren sind,
oder

**variable Komponente:**

| **Inhaltsstoffe** | **Kapselinhalt in mg** |
|---|---|
| Calciumcarbonat | 45,0 |
| Granatapfel-Extrakt | 15,0 |
| Haifischknorpelpulver | 25,0 |

| | |
|---|---|
| Microcristalline Cellulose: 15 mg | |

Diese Zusammensetzung eignet sich bevorzugt für Personen, die unter dem Sternzeichen Steinbock geboren sind,
oder

**variable Komponente:**

| **Inhaltsstoffe** | **Kapselinhalt in mg** |
|---|---|
| Zink-Citrat - 3 Hydrat (32 % Zn) | 50,0 |
| Natriumselenat (0,1 % Selen) | 10,0 |
| Papain | 20,0 |

| | |
|---|---|
| Microcristalline Cellulose: 20 mg | |

Diese Zusammensetzung eignet sich bevorzugt für Personen, welche unter dem Sternzeichen Stier geboren sind,
oder

**variable Komponente:**

| **Inhaltsstoffe** | **Kapselinhalt in mg** |
|---|---|
| Papain | 20,0 |
| Bromealin (2000 GDU) | 15,0 |
| Rosmarinblattpulver | 55,0 |

| | |
|---|---|
| Microcristalline Cellulose: 10 mg | |

Diese Zusammensetzung eignet sich insbesondere für Personen, die unter dem Sternzeichen Waage geboren sind,
oder

**variable Komponente:**

| **Inhaltsstoffe** | **Kapselinhalt in mg** |
|---|---|
| L-Arginin | 45,0 |
| Granatapfel-Extrakt | 20,0 |
| Melisse | 25,0 |

| | |
|---|---|
| Microcristalline Cellulose: 10 mg | |

Diese Rezeptur eignet sich insbesondere für Personen, welche unter dem Sternzeichen Wassermann geboren sind,
oder

**variable Komponente:**

| **Inhaltsstoffe** | **Kapselinhalt in mg** |
|---|---|
| Granatapfel-Extrakt | 50,0 |
| Taurin | 20,0 |
| L-Glutamin | 20,0 |

| | |
|---|---|
| Microcristalline Cellulose: 10 mg | |

Diese Zusammensetzung eignet sich insbesondere für Personen, die unter dem Sternzeichen Widder geboren sind,
oder

**variable Komponente:**

| **Inhaltsstoffe** | **Kapselinhalt in mg** |
|---|---|
| Zink-Citrat - 3 Hydrat (32 % Zn) | 50,0 |
| Curcumin | 10,0 |
| Hibiskus-Anthozyane | 20,0 |

| | |
|---|---|
| Microcristalline Cellulose: 20 mg | |

Diese Zusammensetzung eignet sich insbesondere für Personen, welche unter dem Sternzeichen Zwilling geboren sind,
oder

**variable Komponente:**

| **Inhaltsstoffe** | **Kapselinhalt in mg** |
|---|---|
| Zink-Citrat - 3 Hydrat (32 % Zn) | 25,0 |
| Natriumselenat (0,1 % Selen) | 10,0 |
| Maütake-Pilzextrakt | 40,0 |

| | |
|---|---|
| Microcristalline Cellulose: 25 mg | |

Diese Zusammensetzung eignet sich besonders für Personen, welche unter dem Sternzeichen Fische geboren sind,
oder

**variable Komponente:**

| **Inhaltsstoffe** | **Kapselinhalt in mg** |
|---|---|
| Calciumcarbonat | 40,0 |
| Bromealin | 10,0 |
| Taurin | 30,0 |

| | |
|---|---|
| Microcristalline Cellulose: 20 mg | |

Diese Zusammensetzung eignet sich insbesondere für Personen, die unter dem Sternzeichen Jungfrau geboren sind.

## Patentansprüche

1. Nahrungsergänzungsmittel umfassend einen Gemüsebestandteil, einen Grasbestandteil, einen Fruchtbestandteil und ein Vitamin, und optional ein Antioxidationsmittel, und/oder ein Coenzym, und/oder ein Kräuterbestandteil, und/oder ein Fischbestandteil, und/oder Nussbestandteil, und/oder ein Mineral, und/oder ein Protein, und/oder eine Aminosäure, und/oder Zink-Citrat, und/oder Curcumin, und/oder L-Carnitin, und/oder Taurin.

2. Nahrungsergänzungsmittel nach Anspruch 1, wobei das Vitamin ausgewählt ist aus der Gruppe bestehend aus Vitamin C, D-alpha-Tocopherol, Vitamin B1, Vitamin B2, Niacin, Pantothensäure, Vitamin B6, Vitamin B12, Folsäure, Biotin, Vitamin A, Vitamin D und Vitamin K.

3. Nahrungsergänzungsmittel nach Anspruch 1 oder 2, wobei das Protein ausgewählt ist aus der Gruppe bestehend aus Papain, Bromealin, Albumin, Collagen, Milchprotein, Molkeprotein und Eiprotein.

4. Nahrungsergänzungsmittel nach einem der Ansprüche 1-3, wobei die Aminosäure ausgewählt ist aus der Gruppe bestehend aus L-Arginin, L-Glutamin, L-Alanin, L-Asparagin, L-Asparaginsäure, L-Cystein, L-Glutaminsäure, Glycin, L-Histidin, L-Isoleucin, L-Leucin, L-Lysin, L-Methionin, L-Phenylalanin, L-Prolin, L-Serin, L-Threonin, L-Tryptophan, L-Tyrosin, L-Valin, L-Selenocystein und L-Pyrrolysin.

5. Nahrungsergänzungsmittel nach einem der Ansprüche 1-4, wobei eine Grundkomponente einen Karottenbestandteil, einen Gerstengrasbestandteil, einen Ananasbestandteil, einen schwarze Johannisbeerbestandteil, Calcium-Ascorbat, D-alpha-Tocopherol, Vitamin B1, Vitamin B2, Niacin, Pantothensäure, Vitamin B6, Vitamin B12, Folsäure, Biotin und oligomeres Procyanidin umfasst, und eine variable Komponente ausgewählt ist aus der Gruppe bestehend aus Kürbis, Melisse, Shitake Pilz, Enzian, Wolfsbeere, Rosmarin, Hibiskus, Granatapfel, Maitake Pilz, Haifischknorpel, Calciumcarbonat, Natriumselenat, Papain, Bromealin, L-Arginin, L-Glutamin, Zink-Citrat, Curcumin, L-Carnitin und Taurin.

6. Nahrungsergänzungsmittel nach Anspruch 5, wobei die variable Komponente 1-10, bevorzugt 1-8, besonders bevorzugt 1-5, insbesondere bevorzugt 1-3 Bestandteile ausgewählt aus der Gruppe bestehend aus Kürbis, Melisse, Shitake Pilz, Enzian, Wolfsbeere, Rosmarin, Hibiskus, Granatapfel, Maitake Pilz, Haifischknorpel, Calciumcarbonat, Natriumselenat, Papain, Bromealin, L-Arginin, L-Glutamin, Zink-Citrat, Curcumin, L-Carnitin und Taurin umfasst.

7. Nahrungsergänzungsmittel nach einem der Ansprüche 1-6, wobei bezogen auf das Gewicht des Nahrungsergänzungsmittels der Anteil des Gemüsebestandteils 12-30%, des Grasbestandteils 5-15%, des Fruchtbestandteils 15-35%, des Vitamins 20-25%, des Antioxidationsmittels 2-19%, des Kräuterbestandteils 2-25%, des Fischbestandteils 2-7%, des Minerals 1-10%, des Proteins 1-6%, der Aminosäure 2-12%, des Zink-Citrats 4-15%, des Curcumins 1-8%, des L-Carnitins 4-8% und des Taurins 3-8% beträgt.

8. Verfahren zur Herstellung eines Nahrungsergänzungsmittels nach einem der Ansprüche 1-7, wobei die Bestandteile mit einem pharmazeutischen Träger versehen werden.

9. Verfahren zur Herstellung eines Nahrungsergänzungsmittels nach Anspruch 8, wobei der pharmazeutischen Träger ausgewählt ist aus der Gruppe bestehend aus microcristalliner Cellulose, Carboxymethylcellulose, Alginat, Agarose, Alkohol, Wasser, Kochsalzlösung, Öl, Milchzucker und Paraffin.

10. Verwendung des Nahrungsergänzungsmittels nach einem der Ansprüche 1-7 zur Herstellung eines Medikaments zur Verbesserung des allgemeinen Gesundheitszustands.
